# EUROPÄISCHE PATENTSCHRIFT

(11) **EP 2 252 244 B1**
(45) Veröffentlichungstag und Bekanntmachung des Hinweises auf die Patenterteilung: **20.06.2012**
(21) Anmeldenummer: 09723428.0
(22) Anmeldetag: 30.01.2009
(51) Int. Cl.: A61F 5/56, A61B 5/0205, A61B 5/08

(54) **MEDIZINISCHE DETEKTIONSVORRICHTUNG ZUR DETEKTION VON SCHLAFAPNOE UND/ODER SCHLAFHYPOPNOEN**
MEDICAL DETECTION DEVICE FOR DETECTING SLEEP APNEA AND/OR SLEEP HYPOPNEA
DISPOSITIF MÉDICAL DE DÉTECTION DESTINÉ À DÉTECTER L APNÉE DU SOMMEIL ET/OU LES HYPOPNÉES DU SOMMEIL

(30) Priorität: 17.03.2008 DE 102008014652
(43) Veröffentlichungstag der Anmeldung: 24.11.2010
(73) Patentinhaber: Robert Bosch GmbH, 70442 Stuttgart (DE)
(72) Erfinder: ESCHLER, Johannes, 71254 Ditzingen (DE); BAEHREN, Thomas, 71686 Remseck A.N. (DE); LADSTAETTER, Ulrich, 71384 Weinstadt (DE); PAULS, Angelika, 70197 Stuttgart (DE)
(86) Internationale Anmeldenummer: PCT/EP2009/051088
(87) Internationale Veröffentlichungsnummer: WO 2009/115366

(56) Entgegenhaltungen:
- EP-A- 0 371 424
- EP-A- 0 647 441
- DE-U1- 20 106 456
- FR-A- 2 847 796
- US-A1- 2004 039 254

## Beschreibung

### Stand der Technik

Die obstruktive Schlafapnoe ist die am häufigsten vorkommende Form der schlafbezogenen Atemstörungen. Allein in Deutschland leiden schätzungsweise mehr als 8% der Bevölkerung an dieser Erkrankung und damit auch an ihren Folgen. Von diesen 8% Betroffenen ist nur bei etwa 5% die Erkrankung diagnostiziert und wird therapiert.

Bei der obstruktiven Schlafapnoe tritt für eine längere Zeitdauer ein vollständiger Atemstillstand aufgrund eines kompletten Verschlusses der oberen Atemwege auf. Eine Apnoe ist per Definition ein Atemstillstand von mehr als 10 Sekunden Dauer. Eine weitere Form der Atemstörung ist die Hypopnoe. Hier kommt es zu einem 50%igen Ausfall im Luftstrom. Lang andauernde Atemstillstände oder Hypopnoen wiederholen sich des Öfteren, so dass der Schlaf des Betroffenen stark fragmentiert wird. Die Folgen der obstruktiven Schlafapnoe sind kurzfristig Tagesschläfrigkeit und verminderte Leistungsfähigkeit; langfristig kann die obstruktive Schlafapnoe zu Bluthochdruck, zu einem erhöhten Herzinfarktrisiko, zu Impotenz, etc. führen.

In der Praxis folgt die Verdachtsdiagnose in der Regel relativ spät durch erhöhten Leidensdruck des Betroffenen bei einer ausführlichen Anamnese beim Hausarzt. Wird ein nächtlicher Atemstillstand vermutet, erfolgt die endgültige Befundung und Differentialdiagnose durch eine zeit- und kostenintensive polysomnographische Überwachung im Schlaflabor.

Aus der EP 1 797 846 A1 sind ein Verfahren und eine Vorrichtung zum Behandeln von Schlafapnoe bekannt. Die Vorrichtung ist derart ausgebildet, dass diese eine Kraft auf den Hals des Benutzers ausübt, derart, dass der Hals zum Verhindern eines Verschlusses der Atemwege überstreckt wird.

Aus der EP 37 14 24 A1, die als nächster Stand der Technik angesehen wird, ist eine Überwachungsvorrichtung zur Diagnose von Apnoe bekannt, bei der ein ambulantes Diagnose-Aufzeichnungsgerät, Mittel zur Abtastung der Herzspannung, Mittel zur Erfassung der Herzfrequenz aufgrund dieser Herzspannung, Mittel zur Erfassung von Atmungslauten, sowie Mittel zur Erfassung von Schnarchlauten umfasst. Ferner sind Mittel zur Aufzeichnung kodierter Signale vorgesehen, welche die Herzfrequenz und die Atmungs- und Schnarchlaute darstellen.

Aus der FR 28 47 796 A1 ist eine Vorrichtung zur Atmungsüberwachung eines Patienten bekannt, wenn die Atmungsfrequenz eine vorgegebene Grenze unterschreitet, wird ein Warnsignal ausgegeben.

Die DE 20106456 U1 beschreibt eine Schnarchwarnvorrichtung, bei der in einem Gehäuse ein Mikrofon zum detektieren des Schnarchgeräusches eines Benutzers vorgesehen ist. Ferner ist eine Lampe bzw. eine Vibrationseinrichtung zur Warnung des Benutzers vorgesehen.

Die EP 06 47 441 A1 beschreibt ein Halsband mit elektronischer Baueinheit, die durch Akustik das Schnarchen verhindert. Wird ein Schnarchen empfangen, so wird hierzu ein Summer ausgelöst.

### Offenbarung der Erfindung

### Technische Aufgabe

Der Erfindung liegt die Aufgabe zugrunde, einen kostengünstigen Selbsttest für die Heimanwendungen zur Vorabklärung von Atemstörungen, insbesondere von Apnoen und/oder Hypopnoen, vorzuschlagen.

### Technische Lösung

Diese Aufgabe wird mit einer medizinischen Detektionsvorrichtung mit den Merkmalen des Anspruchs 1 gelöst. Vorteilhafte Weiterbildungen der Erfindung sind in den Unteransprüchen angegeben. In den Rahmen der Erfindung fallen sämtliche Kombinationen aus zumindest zwei von in der Beschreibung, den Ansprüchen und/oder den Figuren offenbarten Merkmalen.

Der Erfindung liegt der Gedanke zugrunde, eine bevorzugt als Einwegartikel ausgebildete, medizinische Detektionsvorrichtung zur Detektion von Schlafapnoe vorzuschlagen, die zum einen Fixiermittel aufweist, mit denen die Detektionsvorrichtung am menschlichen Körper, insbesondere im Halsbereich oder im Thoraxbereich, befestigbar ist und die andererseits mindestens ein Mikrofon zum Erfassen von Atemgeräuschen aufweist, die mit integralen Logikmitteln der Detektionsvorrichtung im Hinblick auf das Auftreten von Atemstörungen, insbesondere von Schlafapnoen und/oder Hypopnoen auswertbar ist. Anders ausgedrückt sind die Logikmittel der medizinischen Detektionsvorrichtung derart ausgebildet, dass mit ihnen eine Signalanalyse der von dem Mikrofon erfassten Signale (Atemgeräusche) durchführbar ist, so dass die medizinische Detektionsvorrichtung als Selbsttest für die Heimanwendung zur Vorabklärung von Atemstörungen einsetzbar ist. Bevorzugt wird die medizinische Detektionsvorrichtung vor dem Zubettgehen mit Hilfe der Fixiermittel am Körper, insbesondere im Halsbereich und/oder im Thoraxbereich, fixiert, woraufhin die Detektionsvorrichtung, ggf. nach einer manuellen Aktivierung, ihre Analyse-, bzw. Auswertetätigkeit automatisch ausführt. Von besonderem Vorteil ist eine Ausführungsform, bei der das mindestens eine Mikrofon als Micro-Electro-Mechanical-System (MEMS-Bauteil) ausgebildet ist, um zum einen das Gewicht der Detektionsvorrichtung zu minimieren und zum anderen das Tragen der Detektionsvorrichtung für den Benutzer so angenehm wie möglich zu gestalten. Alternativ aufgebaute Miniaturmikrofone sind zusätzlich oder alternativ einsetzbar.

In Weiterbildung der Erfindung ist mit Vorteil vorgesehen, dass die Fixiermittel ein Heftpflaster umfassen und/oder als Heftpflaster ausgebildet sind. Anders ausgedrückt ist eine Ausführungsform bevorzugt, bei der das mindestens eine signalleitend mit den Logikmitteln verbundene Mikrofon sowie die Logikmittel in ein Heftpflaster integriert sind, welches vom Benutzer auf einfache Weise an eine vorgegebene Körperstelle aufgeklebt und nach der Auswertung wieder entfernt werden kann.

Von besonderem Vorteil ist eine Ausführungsform, bei der die Logikmittel derart ausgebildet sind, dass mit ihnen die Atemgeräusche im Wege einer Signalanalyse im Hinblick auf Atemstörungen, insbesondere im Hinblick auf Atemstillstände, auswertbar sind, die über einen Mindestzeitraum, beispielsweise über einen Zeitraum von 10 Sekunden andauert. Bevorzugt werden dabei nur Apnoen und/oder Hypopnoen gewertet bzw. gezählt, die über den vorgegebenen oder vorgebbaren Mindestzeitraum andauern. Hierzu erfassen die Logikmittel bevorzugt einen Atemstörungszähler, der, wenn eine über den Mindestraum andauernde Atemstörung von den Logikmitteln festgestellt wird, um einen Wert erhöht wird.

Von besonderem Vorteil ist eine Ausführungsform, bei der die Herztätigkeit des Betroffenen, insbesondere die Herzfrequenz, zur Plausibilisierung von potentiellen Atemstörungen von den Logikmitteln berücksichtigt wird. Dieser speziellen Ausbildung der Logikmittel liegt die Erkenntnis zugrunde, dass es bei einer länger andauernden Atemstörung, insbesondere Atemstillständen, aufgrund der Sauerstoffunterversorgung des Körpers zu einer Steigerung der Herzfrequenz kommt. Setzt der Atem wieder ein, normalisiert sich die Herzfrequenz wieder. Bevorzugt wird der Atemstörungszähler nur dann um einen Wert erhöht, wenn die Herztätigkeit, insbesondere die Herzfrequenz, während der potenziellen Atemstörung, also während der atemgeräuschauffälligen, insbesondere atemgeräuschfreien, Zeit, einen bestimmten Wert, beispielsweise einen festen Wert oder einen gleitenden Mittelwert, überschreitet.

In Weiterbildung der Erfindung ist mit Vorteil vorgesehen, dass die Logikmittel zur Überwachung bzw. Auswertung der Herztätigkeit die Herzgeräusche auswerten, die über das mindestens eine Mikrofon erfasst werden. Anders ausgedrückt werden bei dem von den Logikmitteln durchgeführten Signalanalyseverfahren bevorzugt zusätzlich zu den Atemgeräuschen die Herzgeräusche, insbesondere im Hinblick auf die Herzfrequenz, zur Plausibilisierung von potenziellen Atemstörungen ausgewertet.

Wie zuvor erwähnt, ist eine Ausführungsform bevorzugt, bei der die Logikmittel die Anzahl der Atemstörungen zählen, die über eine Mindestzeitdauer andauern. Bevorzugt sind die Logikmittel derart ausgebildet, dass sie eine Atemstörung nur dann als Apnoe und/oder Hypopnoe werten, wenn während der atemgeräuschauffälligen, insbesondere atemgeräuschfreien, Zeit die Herzfrequenz über einen bestimmten Wert ansteigt.

Erfindungsgemäß ist es vorteilhaft, wenn den Logikmitteln Ausgabemittel zur Ausgabe eines Auswertungsergebnisses zugeordnet sind. So ist es beispielsweise möglich, die Ausgabemittel derart auszubilden, dass diese anzeigen, ob eine Mindestanzahl von Atemstörungen, beispielsweise eine Atemstörung oder fünf Atemstörungen, während des Schlafes aufgetreten sind. Die Ausgabemittel können beispielsweise auch derart ausgebildet sein, dass diese die Auskunft über die Anzahl der vorgelegenen, vorzugsweise plausibilisierten, Apnoen und/oder Hypopnoen geben. So können beispielsweise je nach finaler Größe des Wertes des Atemstörungszählers unterschiedliche Ausgänge der Logikmittel aktiviert werden, woraus unterschiedliche Ausgaben resultieren.

Zusätzlich oder alternativ können die Ausgabemittel Funkmittel umfassen, über die das Auswertungsergebnis und/oder der Stand des Atemstörungszählers drahtlos abgerufen werden kann. Zusätzlich oder alternativ zu Funkmitteln ist es denkbar, einen Anschluss zum kabelgestützten Auslesen des Auswertungsergebnisses und/oder des Atemstörungszählers vorzusehen.

Von besonderem Vorteil ist eine Ausführungsform der medizinischen Detektionsvorrichtung, bei der die Ausgabemittel ein Auswertungsergebnis auf Basis einer elektrochemischen Reaktion optisch visualisierend ausgebildet sind. Hierdurch kann der Energiebedarf der Ausgabemittel minimiert werden. So kann beispielsweise durch die an einem Ausgang der Logikmittel anliegende Spannung, die beispielsweise abhängig von der Anzahl der festgestellten, insbesondere plausibilisierten Atemstörungen, ist, eine elektrochemische Farbreaktion ausgelöst werden, die einen bestimmten Bereich der Detektionsvorrichtung, insbesondere des Heftpflasters, vorzugsweise irreversibel, verfärbt. In einer weiteren Variante können, je nach finaler Größe des Wertes des Atemstörungszählers, unterschiedliche Ausgänge der Logikmittel aktiviert werden, die dann in unterschiedlichen Bereichen der Detektionsvorrichtung, insbesondere des Heftpflasters, Farbreaktion auslösen und somit die Detektionsvorrichtung, vorzugsweise irreversibel, an den unterschiedlichen Bereichen verfärben. Beispielsweise kann über eine durch eine elektrochemische Reaktion sichtbar gemachte Beschriftung, insbesondere auf dem Heftpflaster, Auskunft über die Anzahl der, insbesondere plausibilisierten, Atemstörungen, insbesondere Apnoen und/oder Hypopnoen, gegeben werden.

Von besonderem Vorteil ist eine Ausführungsform, bei der die Detektionsvorrichtung eine integrale Spannungsquelle aufweist, mit der die Logikmittel und/oder das Mikrofon und/oder die Ausgabemittel mit der benötigten elektrischen Energie versorgbar sind. Besonders bevorzugt ist es, wenn die Spannungsquelle mindestens einen Kondensator oder eine Kondensatoranordnung umfasst, so dass die Detektionsvorrichtung nach Gebrauch ohne eine Gefährdung für die Umwelt entsorgbar ist.

In Weiterbildung der Erfindung ist mit Vorteil vorgesehen, dass das Mikrofon derart in der Detektionsvorrichtung, insbesondere im Heftpflaster, angeordnet ist, dass dieses bei am Menschen bzw. am Körper fixierter Detektionsvorrichtung unmittelbar auf der Haut, vorzugsweise am Hals, besonders bevorzugt in der Nähe der Arteria Carotis und/oder am Thorax anliegt, um Atemgeräusche und/oder Herzgeräusche erfassen zu können. Es ist auch denkbar, zur Erfassung von Atemgeräuschen und Herzgeräuschen separate Mikrofone einzusetzen.

### Kurze Beschreibung der Zeichnung

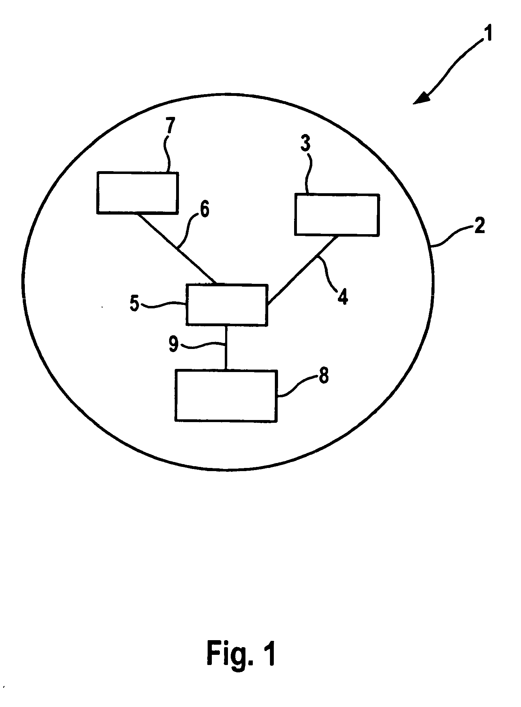

Weitere Vorteile, Merkmale und Einzelheiten der Erfindung ergeben sich aus der nachfolgenden Beschreibung eines bevorzugten Ausführungsbeispiels sowie anhand der Zeichnung. Diese zeigt in der einzigen
- Fig. 1:: eine schematische Darstellung einer medizinischen Detektionsvorrichtung.

### Ausführungsformen der Erfindung

In Fig. 1 ist stark schematisiert eine mögliche Ausführungsform einer medizinischen Detektionsvorrichtung 1 zur Detektion von Schlafatemstörungen gezeigt. Die Detektionsvorrichtung 1 umfasst als Heftpflaster ausgebildete Fixiermittel 2 zum lösbaren Festlegen der Detektionsvorrichtung 1 auf der Haut eines menschlichen Körpers. Das Heftpflaster weist hierzu nicht eingezeichnete, dem Körper zugewandte, Klebebereiche auf.

Die Detektionsvorrichtung 1 umfasst ein als MEMS-Bauteil ausgebildetes Mikrofon 3 zum Aufnehmen, d. h. zum Erfassen, von Atem- und Herzgeräuschen. Das Mikrofon 3 ist über eine Signalleitung 4 signalleitend mit einen Mikroprozessor umfassenden Logikmitteln 5 verbunden. Die Logikmittel 5 werden über eine Versorgungsleitung 6 mit elektrischer Energie von einer integralen Spannungsquelle 7 der Detektionsvorrichtung 1 versorgt, wobei die elektrische Energie von den Logikmitteln 5 zur Versorgung des Mikrofons 3 und von signalleitend mit den Logikmitteln 5 verbundenen Ausgabemitteln 8 weitergeleitet.

Die Logikmittel 5 sind zum Auswerten der mittels des Mikrofons 3 erfassten Atemgeräusche und zur Überwachung bzw. Auswertung der ermittelten Herzgeräusche ausgebildet. Wird über einen vorgegebenen Zeitraum, beispielsweise zehn Sekunden, kein Atemgeräusch oder ein auffälliges Atemgeräusch detektiert, so erkennen die Logikmittel 5 eine potenzielle Schlafatemstörung. Steigt gleichzeitig während der atemgeräuschauffälligen Zeit die aus den Herzgeräuschen von den Logikmitteln 5 abgeleitete Herzfrequenz über einen vorgegebenen Wert, beispielsweise einen festen Wert oder einen gleitenden Mittelwert, an, so ist die Schlafapnoe plausibilisiert, und ein integraler Atemstörungszähler der Logikmittel 5 wird um einen Wert erhöht. Bei Bedarf kann auch auf die Auswertung der Herzgeräusche und damit auf eine Plausibilisierung von potenziellen Schlafapnoen verzichtet werden. In diesem Fall wird der Atemstörungszähler bevorzugt bereits dann um einen Wert erhöht, wenn eine Apnoe und/oder Hypopnoe über eine vorgegebene Zeitdauer andauert.

Wie erwähnt, sind die Logikmittel 5 mit den Ausgabemitteln 8 signalleitend verbunden. Hierzu dient eine Signalleitung 9. Bei einer sehr einfachen Ausführungsvariante wird auf die Signalleitung 9 von den Logikmitteln 5 eine Spannung geschaltet, sobald der Atemstörungszähler einen bestimmten Wert, beispielsweise 1, 5 oder 10, überschreitet. Diese Spannung aktiviert die Ausgabemittel 8 derart, dass diese eine elektrochemische Farbreaktion auslösen, die einen bestimmten Bereich der als Heftpflaster ausgebildeten Fixiermittel 2 irreversibel verfärbt. In einer alternativen Variante können, in Abhängigkeit der Größe des Wertes des Atemstörungszählers unterschiedliche Ausgänge der Logikmittel 5, insbesondere des Mikroprozessors, aktiviert werden, die mit unterschiedlichen Farbreaktionsbereichen verbunden sind, so dass je nach aktiviertem Ausgang unterschiedliche Bereiche der Signalmittel verfärbt werden. Die Anzahl der detektierten Schlafatemstörungen oder ein Anzahlbereich kann über eine Farbkodierung oder eine Beschriftung für den Benutzer visualisiert werden.

## Patentansprüche

1. Medizinische Detektionsvorrichtung (1) zur Detektion von Schlafatemstörungen, mit Fixiermitteln (2) zum Festlegen der Detektionsvorrichtung (1) am menschlichen Körper, mit mindestens einem Mikrofon (3) zum Erfassen von Atemgeräuschen und mit zum Auswerten der Atemgeräusche ausgebildeten Logikmitteln (5), **dadurch gekennzeichnet, dass** den Logikmitteln (5) Ausgabemittel (8) zur Ausgabe eines Auswertungsergebnisses zugeordnet sind.

2. Detektionsvorrichtung nach Anspruch 1,
**dadurch gekennzeichnet,**
**dass** die Fixiermittel (2) ein Heftpflaster umfassen.

3. Detektionsvorrichtung nach einem der Ansprüche 1 oder 2,
**dadurch gekennzeichnet,**
**dass** die Logikmittel (5) die Atemgeräusche im Hinblick auf das Auftreten von Apnoen und/oder Hypopnoen auswertend ausgebildet sind, die über einen Mindestzeitraum andauern.

4. Detektionsvorrichtung nach Anspruch 3,
**dadurch gekennzeichnet,**
**dass** die Logikmittel (5) die Herztätigkeit, insbesondere die Herzfrequenz, zur Plausibilisierung von Atemstörungen, insbesondere von Atemstillständen, berücksichtigend ausgebildet ist.

5. Detektionsvorrichtung nach Anspruch 3,
**dadurch gekennzeichnet,**
**dass** die Logikmittel (5) die Herztätigkeit anhand von, insbesondere über das Mikrofon (3) erfassten, Herzgeräuschen überwachend ausgebildet ist.

6. Detektionsvorrichtung nach einem der Ansprüche 3 bis 5,
**dadurch gekennzeichnet,**
**dass** die Logikmittel (5) die, insbesondere plausibilisierten, eine Mindestzeitdauer andauernden, Atemstörungen zählend ausgebildet ist.

7. Detektionsvorrichtung nach einem der vorhergehenden Ansprüche, **dadurch gekennzeichnet,**
**dass** die Ausgabemittel (8) ein Auswertungsergebnis auf Basis einer elektrochemischen Reaktion darstellend ausgebildet sind.

8. Detektionsvorrichtung nach einem der vorhergehenden Ansprüche,
**dadurch gekennzeichnet,**
**dass** eine Spannungsquelle (7), insbesondere mindestens ein Kondensator, vorgesehen ist.

9. Detektionsvorrichtung nach einem der vorhergehenden Ansprüche,
**dadurch gekennzeichnet,**
**dass** das Mikrofon (3) derart angeordnet ist, dass dieses bei am Körper fixierter Detektionsvorrichtung (1) unmittelbar auf der Haut, vorzugsweise am Hals, besonders bevorzugt in der Nähe der Arteria Carotis, und/oder am Thorax, anliegt.

## Claims

1. Medical detection device (1) for detecting breathing disorders during sleep, with fixation means (2) for attaching the detection device (1) to the human body, with at least one microphone (3) for capturing breathing noises and with a logic means (5) configured to evaluate the breathing noises, **characterized in that** output means (8) for outputting an evaluation result are assigned to the logic means (5).

2. Detection device according to Claim 1,
**characterized**
**in that** the fixation means (2) comprise an adhesive plaster.

3. Detection device according to either of Claims 1 and 2,
**characterized**
**in that** the logic means (5) are configured to evaluate the breathing noises in respect of the occurrence of apnoeas and/or hypopnoeas with a duration greater than a minimum period of time.

4. Detection device according to Claim 3,
**characterized**
**in that** the logic means (5) is configured to take the cardiac activity, more particularly the heart rate, into account for determining the plausibility of breathing disorders, more particularly respiratory arrests.

5. Detection device according to Claim 3,
**characterized**
**in that** the logic means (5) is configured to monitor the cardiac activity on the basis of heart noises, which are captured, in particular, by the microphone (3).

6. Detection device according to one of Claims 3 to 5,
**characterized**
**in that** the logic means (5) is configured to count the breathing disorders, more particularly those that have been determined to be plausible, with a duration of a minimum time duration.

7. Detection device according to one of the preceding claims,
**characterized**
**in that** the output means (8) are configured to display an evaluation result on the basis of an electrochemical reaction.

8. Detection device as claimed in one of the preceding claims,
**characterized**
**in that** provision is made for a voltage source (7), more particularly at least one capacitor.

9. Detection device as claimed in one of the preceding claims,
**characterized**
**in that** the microphone (3) is arranged such that when the detection device (1) is affixed on the body said microphone rests directly on the skin, preferably on the neck, particularly preferably in the vicinity of the carotid artery, and/or on the thorax.

## Revendications

1. Dispositif médical de détection (1) servant à détecter des troubles respiratoires du sommeil, avec des moyens de fixation (2) utilisés pour fixer le dispositif de détection (1) au corps humain, avec au moins un microphone (3) utilisé pour enregistrer les bruits de respiration et avec des moyens logiques (5) conçus pour analyser les bruits de respiration, **caractérisé en ce que** des moyens d'émission (8) sont associés aux moyens logiques (5) pour envoyer un résultat d'analyse.

2. Dispositif de détection selon la revendication 1, **caractérisé en ce que** les moyens de fixation (2) comprennent un sparadrap.

3. Dispositif de détection selon l'une quelconque des revendications 1 ou 2, **caractérisé en ce que** les moyens logiques (5) sont conçus pour analyser les bruits de respiration à la recherche d'apnées et/ou d'hypopnées durant plus longtemps qu'une durée minimale.

4. Dispositif de détection selon la revendication 3, **caractérisé en ce que** les moyens logiques (5) sont conçus pour prendre en compte l'activité cardiaque, notamment la fréquence cardiaque, pour étudier la plausibilité de troubles respiratoires, notamment d'arrêts respiratoires.

5. Dispositif de détection selon la revendication 3, **caractérisé en ce que** les moyens logiques (5) sont conçus pour surveiller l'activité cardiaque à l'aide notamment des bruits du coeur enregistrés par le microphone (3).

6. Dispositif de détection selon l'une quelconque des revendications 3 à 5, **caractérisé en ce que** les moyens logiques (5) sont conçus pour compter les troubles respiratoires, notamment ceux détectés comme plausibles et durant plus qu'une durée minimale.

7. Dispositif de détection selon l'une quelconque des revendications précédentes, **caractérisé en ce que** les moyens d'émission (8) sont conçus pour représenter un résultat d'analyse sur la base d'une réaction électrochimique.

8. Dispositif de détection selon l'une quelconque des revendications précédentes, **caractérisé en ce qu'**une source d'alimentation en tension (7) est prévue, notamment au moins un condensateur.

9. Dispositif de détection selon l'une quelconque des revendications précédentes, **caractérisé en ce que** le microphone (3) est disposé de telle sorte qu'il repose, dans le cas d'un dispositif de détection (1) fixé sur le corps, directement sur la peau, de préférence sur le cou, de façon particulièrement préférée à proximité de l'artère carotide et/ou au niveau du thorax.
